# FASCICULE DE BREVET EUROPEEN

(11) **EP 3 264 981 B1**
(45) Date de publication et mention de la délivrance du brevet: **28.07.2021**
(21) Numéro de dépôt: 16714999.6
(22) Date de dépôt: 03.03.2016
(51) Int. Cl.: A61B 5/11, A61B 5/00

(54) **DISPOSITIF D'ASSISTANCE DESTINÉ À UNE PERSONNE ATTEINTE DE TROUBLES MOTEURS D'ORIGINE NEUROLOGIQUE**
HILFSVORRICHTUNG FÜR PERSONEN MIT MOTORISCHEN STÖRUNGEN NEUROLOGISCHEN URSPRUNGS
ASSISTANCE DEVICE INTENDED FOR A PERSON SUFFERING MOTOR DISORDERS OF NEUROLOGICAL ORIGIN

(30) Priorité: 03.03.2015 FR 1551771
(43) Date de publication de la demande: 10.01.2018
(73) Titulaire: Resilient Innovation, 34080 Montpellier (FR)
(72) Inventeur: TEISSIER, Sébastian, 34080 Montpellier (FR); MIRON, Jordan, 11220 Servies en Val (FR)
(74) Mandataire: Regimbeau
(86) Numéro de dépôt international: PCT/FR2016/050488
(87) Numéro de publication internationale: WO 2016/139428

(56) Documents cités:
- WO-A1-2009/083032
- US-A1- 2009 204 030
- US-A1- 2012 101 411
- US-A1- 2014 372 045

## Description

### DOMAINE TECHNIQUE DE L'INVENTION ET CONTEXTE

La présente invention concerne un dispositif d'assistance à la mobilité destiné à des personnes atteintes de troubles moteurs d'origine neurologique, notamment ceux dus à une maladie neurodégénérative telle que la maladie de Parkinson. Par les termes « troubles moteurs », on entend une perte de contrôle par l'utilisateur, partielle ou totale, des muscles lui permettant de se déplacer. Ces troubles moteurs sont susceptibles d'empêcher la marche de cette personne ou de la faire chuter. Plus précisément, la présente demande porte sur un dispositif d'assistance permettant de déclencher une stimulation auditive de l'utilisateur portant ledit dispositif, lorsqu'un épisode de troubles moteurs risque de survenir ou bien survient, afin de lui permettre d'empêcher la survenue de celui-ci.

Par les termes "origine neurologique", on entend le fait que les troubles moteurs sont causés par une altération, chez la personne atteinte, du système nerveux, plutôt que par des troubles ostéo-articulaires ou musculaires. Les maladies neurodégénératives sont généralement la cause de cette altération, et l'invention s'adresse particulièrement aux personnes qui y sont atteintes, et notamment parmi celles-ci la maladie de Parkinson idiopathique, les syndromes parkinsoniens, l'atrophie multi-systématisée, la démence à corps de Lewy, ou la paralysie supranucléaire progressive, entre autres. D'autres maladies comme la sclérose en plaque peuvent être concernées, ainsi que des altérations accidentelles comme les conséquences d'un accident vasculaire cérébral ou d'un accident ischémique transitoire. La description qui suit sera faire en référence à la maladie de Parkinson idiopathique en tant qu'exemple illustratif non limitatif.

La maladie de Parkinson idiopathique est la deuxième pathologie neurodégénérative après la maladie d'Alzheimer. L'âge d'entrée dans la maladie se situe entre 58 et 62 ans et plus rarement (dans moins de 10% des cas) avant 40 ans. Cette pathologie est caractérisée par une altération du fonctionnement du système des ganglions de la base (putamen, globus pallidus, locus niger et le noyau sous-thalamique). Les ganglions de la base sont des noyaux sous-corticaux dans le cerveau faisant partie d'un circuit moteur (incluant le thalamus, cortex moteur, et cortex préfrontal) responsable surtout du contrôle des mouvements volontaires. Dans la maladie de Parkinson idiopathique, on observe une dépopulation des neurones dopaminergiques du locus niger qui sont en relation avec le striatum (atteinte des faisceaux nigro-striés). L'atteinte nigro-striée entraîne une diminution de l'activation du thalamus ce qui conduit à des difficultés d'initiation des mouvements volontaires. Durant de nombreuses années, la maladie de Parkinson a été considérée uniquement comme une pathologie de la motricité caractérisée par la « triade parkinsonienne ». Celle-ci comprend trois symptômes qui sont le tremblement de repos, la rigidité musculaire et l'akinésie. Au début de la maladie de Parkinson, la symptomatologie est classiquement unilatérale pour se bilatéraliser ensuite, en restant toujours asymétrique.

La triade parkinsonienne engendre des troubles moteurs qui, associés à l'instabilité posturale, génèrent des troubles de la marche et des chutes souvent traumatisantes (fractures du col du fémur, traumatismes crâniens). Le déficit de dopamine au niveau nigro-strié expliquerait en partie ces troubles moteurs. Le sujet âgé normal montre une dégradation de la marche, une diminution de la vitesse de marche, de la longueur des pas, une augmentation de la fréquence des pas et une asymétrie et variabilité des pas. Chez le sujet atteint de syndrome parkinsonien, ces déficits sont beaucoup plus marqués. Parmi les troubles de la marche, on retrouve aussi l'enrayage cinétique (ou "Freezing" pour "Freezing of Gait" en anglais). Il s'agit d'un trouble de la marche défini comme une réduction de la progression du pas malgré une intention du sujet de vouloir avancer, qui mène généralement un arrêt total du sujet *atteint* de la maladie de parkinson, malgré cette intention de vouloir avancer. L'enrayage cinétique est présent chez la très grande majorité des patients à un stade évolué.

L'enrayage cinétique se manifeste lors de l'initiation de la marche (surtout après le lever de la chaise ou du lit ou après une immobilisation prolongée) ou pendant celle-ci favorisé par la situation de double tâche dont le calcul mental, par le stress, les passages étroits, ainsi que les passages de portes. L'enrayage cinétique, ou « freezing », se caractérise par une augmentation de la fréquence de marche, associée à une diminution de la longueur des foulées, mais également par la sensation des pieds « aimantés au sol ». Ce phénomène d'enrayage cinétique est l'un des symptômes les plus invalidants de la maladie de Parkinson venant altérer de façon significative la qualité de vie des patients.

Le phénomène d'enrayage cinétique peut être atténué ou évité en faisant écouter à un patient une pulsation rythmique. Plus précisément, elle consiste en la présentation d'indices auditifs rythmiques (par exemple les sons d'un métronome ou de la musique), afin d'aider le patient à mieux marcher en lui permettant de régulariser, stabiliser ses mouvements, grâce à la synchronisation des mouvements de ses membres avec une musique par exemple.

Le document US 2010/0274304 A1 décrit un exemple d'un type de dispositif, placé sur une chaussure de l'utilisateur et émettant un signal lorsque le dispositif détecte une irrégularité dans la cadence de pas. Plus précisément, le signal est émis à partir du moment où l'utilisateur n'avance plus. Le signal émis est alors transformé en un signal auditif par un récepteur-émetteur placé au niveau de l'oreille de l'utilisateur, pour lui permettre de réenclencher sa marche. Autrement dit, ce dispositif permet à l'utilisateur de réinitialiser sa marche après que celle-ci fut interrompue par un phénomène d'enrayage cinétique. Cependant, une telle détection est imparfaite, car le changement d'allure du marcheur peut être causé par divers facteurs autres qu'un enrayage cinétique. Il en résulte un déclenchement intempestif de la stimulation auditive, qui se révèle gênante et dissuade la personne d'utiliser le dispositif.

Le document US 2012/101411 A1 une méthode de récolte de donnée relatives à l'enrayage cinétique, qui comprend la collecte de données de mouvement, la détermination à partir de ces données d'un paramètre de mouvement incluant une dérivée du troisième ordre de la position, la comparaison du paramètre de mouvement avec une valeur de seuil, et compter au moins une presque chute si le paramètre de mouvement excède une valeur de seuil. Cette approche ne distingue pas entre les postures ni ne prend en compte une durée de franchissement du seuil, ce qui implique le choix d'un seuil peu sensible, empêchant une détection précoce de l'enrayage cinétique.

Le document US 2009/204030 A1 traite d'une méthode de rééducation des patients avec des handicaps moteurs, et en particulier des patients hémiplégiques. L'idée essentielle est d'évaluer l'utilisation fonctionnelle d'un membre en détermination la synchronicité des mouvements du patient en fonction de la cyclicité du mouvement. Une telle approche ne permet pas une détection de l'enrayage cinétique précoce et fiable.

Le document WO 2009/083032 A1 décrit une méthode pour détecter des épisodes de blocage physique d'une activité d'un individu. Cette méthode comprend l'acquisition d'un signal de mouvement d'au moins une partie corporelle de l'individu. Les signaux de mouvement peuvent être des accélérations statiques ou dynamiques, et des vitesses de rotation de cette partie corporelle. Le jerk est ensuite calculé à partir des accélérations statiques ou dynamiques. Le mouvement est considéré comme stable ou non en fonction du fait que le jerk reste ou non en deçà d'un seuil. Cette approche ne distingue pas entre les postures ni ne prend en compte une durée de franchissement du seuil, ce qui implique le choix d'un seuil peu sensible, empêchant une détection précoce de l'enrayage cinétique.

Il existe un besoin pour un dispositif d'assistance à la motricité permettant de prévenir la survenue d'un phénomène d'enrayage cinétique de la marche d'un utilisateur portant ledit dispositif et permettant de détecter de façon précoce un phénomène d'enrayage cinétique de la marche d'un utilisateur portant ledit dispositif, afin de lui permettre de retrouver une marche normale, en évitant cependant un déclenchement intempestif du dispositif en l'absence d'enrayage cinétique.

### PRÉSENTATION DE L'INVENTION

L'invention a pour but de remédier au moins en partie à ces inconvénients et préférentiellement à tous, en proposant un dispositif d'assistance à la motricité destiné à une personne atteinte de troubles moteurs d'origine neurologique afin qu'elle permette d'éviter la survenue d'un enrayage cinétique, et dans le cas d'un enrayage cinétique, de détecter sa survenue au plus vite, sans faux-positif, et qui permette de stimuler auditivement cette personne pour qu'elle retrouve une motricité acceptable.

Il est ainsi proposé un dispositif d'assistance destiné à une personne atteinte de troubles moteurs d'origine neurologique, comprenant :
- un accéléromètre adapté pour être porté par la personne pour mesurer des accélérations selon au moins une première direction qui correspond à l'axe horizontal ; et
- des moyens de mémorisation mémorisant une pluralité de valeurs seuils d'accélération de prévention préalablement définies pour ladite première direction ; et
- des moyens d'émission d'une stimulation auditive perceptible par la personne portant l'accéléromètre; et
- des moyens de calcul connectés à l'accéléromètre, aux moyens de mémorisation et aux moyens d'émission ;
le dispositif d'assistance comprenant des moyens de mémorisation mémorisant une pluralité de valeurs seuils d'accélération d'anticipation préalablement définies pour ladite première direction, chaque valeur seuil d'accélération d'anticipation étant associée à une posture , et les moyens de calcul étant configurés pour :
a) déterminer une posture de la personne à partir des accélérations mesurées,
b) comparer les accélérations mesurées par la centrale inertielle dans la première direction par rapport à au moins une valeur seuil d'accélération d'anticipation associée à ladite posture ;
c) commander l'émission d'une stimulation auditive en fonction du franchissement de ladite valeur seuil d'accélération d'anticipation associée à ladite posture;
le dispositif d'assistance étant caractérisé en ce que les moyens de calcul sont configurés pour :
- associer également chaque posture à une durée déterminée fonction du sens de franchissement de ladite valeur seuil d'accélération d'anticipation par les accélérations mesurées, et
- commander l'émission de la stimulation auditive en fonction du franchissement de ladite valeur seuil d'accélération d'anticipation associée à ladite posture pendant la durée déterminée.

Ce dispositif est avantageusement complété par les caractéristiques suivantes, prises seules ou en quelconque de leurs combinaisons techniquement possibles :
- les postures comprennent la posture assis-couché, et une valeur seuil d'accélération d'anticipation associée à la posture assis-couché correspond à un déséquilibre vers l'arrière ;
- les postures comprennent la posture debout, une valeur seuil d'accélération d'anticipation associée à la posture debout correspondant à un déséquilibre vers l'arrière et une valeur seuil d'accélération d'anticipation associée à la posture debout correspondant à un déplacement vers l'avant ;
- le dispositif comprend une centrale inertielle de laquelle fait partie l'accéléromètre, ladite centrale inertielle étant en outre configurée pour déterminer une orientation du tronc de la personne, ladite orientation étant utilisée pour déterminer la posture de la personne;
- l'accéléromètre est également adaptée pour mesurer des accélérations selon une seconde direction, ladite seconde direction correspondant à la verticale, et les moyens de mémorisation mémorisent au moins une valeur seuil d'accélération d'alerte préalablement définie dans une seconde direction, les moyens de calculs étant en outre configurés pour :
   a) comparer les accélérations mesurées par la centrale inertielle dans la seconde direction,
   b) générer un signal d'alerte lorsqu'une accélération mesurée a une valeur supérieure à la valeur seuil d'accélération d'alerte,
   c) commander aux moyens d'émission d'émettre une stimulation auditive en fonction du signal d'alerte ;
- les moyens de mémorisation mémorisent une valeur seuil de temps et une valeur seuil d'accélération de marche, et les moyens de calculs sont configurés pour mesurer un intervalle de temps entre deux mesures d'accélérations dépassant ladite valeur seuil d'accélération de marche et déclencher une alerte lorsqu'un intervalle de temps entre deux dépassements consécutifs de ladite valeur seuil d'accélération de marche est inférieur à la valeur seuil de temps mémorisée ;
- les moyens de mémorisation mémorisent une période de référence correspondant à la valeur seuil de temps, et en ce que les moyens de calcul commandent aux moyens d'émission d'émettre la stimulation auditive selon la période de référence ;
- les moyens de calcul sont configurés pour commander aux moyens d'émission d'émettre une stimulation auditive à partir du moment où au moins deux signaux d'alerte sont générés dans un intervalle de temps inférieur à 0,5 seconde, de préférence inférieur à 0,3 seconde ;
- le dispositif comprend :
   - des moyens de mesure du rythme cardiaque adaptés à être portés par la personne pour mesurer son rythme cardiaque ; et
   - les moyens de mémorisation mémorisent une valeur seuil de rythme cardiaque ; et les moyens de calculs sont configurés pour générer un signal d'alerte lorsque le rythme cardiaque mesuré a une valeur supérieure à la valeur seuil de rythme cardiaque mémorisée ;
- l'accéléromètre est configuré pour mesurer une accélération selon une autre direction différente de celle de la seconde direction, et les moyens de mémorisation mémorisent une seconde valeur seuil d'accélération, et les moyens de calculs sont configurés pour inhiber la génération d'un signal d'alerte pendant une durée prédéterminée lorsqu'une accélération mesurée dans l'autre direction a une valeur supérieure à la seconde valeur seuil d'accélération mémorisée ;
- les moyens de calcul sont configurés pour déterminer à partir des mesures d'accélération mesurées par l'accéléromètre, l'orientation du tronc de la personne ;
- les moyens de mémorisation mémorisent une valeur seuil d'accélération marche et une valeur de temps repos, et les moyens de calcul détectent une position assise ou couchée de la personne portant l'accéléromètre lorsque toutes les valeurs d'accélération mesurées par l'accéléromètre sont inférieures à la valeur seuil d'accélération marche pendant une durée supérieure à la valeur de temps repos ;
- les moyens de calcul sont configurés pour comparer les accélérations mesurées par les accéléromètres de façon périodique, la valeur de cette période étant égale à :
- une première période de mesure lorsque le tronc de la personne est horizontal ; et/ou
- une deuxième période de mesure inférieure à la première valeur, lorsque le tronc de la personne est vertical ; et
- une troisième période de mesure inférieure à la deuxième valeur, lorsque la personne marche.

L'invention concerne également un procédé de calibration d'un dispositif d'assistance selon l'une des revendications précédentes, comprenant les étapes selon lesquelles :
- l'accéléromètre est disposé sur la personne,
- une série de mesures d'accélération est acquise pendant que la personne marche,
- la série est amputée des parties de la série de mesures d'accélération correspondant à une motricité altérée de la personne,
- les valeurs d'accélération d'anticipation et/ou une valeur seuil d'accélération d'alerte est déterminée à partir de la moyenne des accélérations de la série amputée.

De préférence, la valeur seuil d'accélération de marche et/ou la valeur seuil de temps est déterminée à partir de la moyenne des accélérations de la série amputée.

### PRÉSENTATION DES FIGURES

L'invention sera mieux comprise, grâce à la description ci-après, qui se rapporte à des modes de réalisations et des variantes selon la présente invention, donnés à titre d'exemples non limitatifs et expliqués avec référence aux dessins schématiques annexés, dans lesquels :
- la figure 1 est un schéma illustrant une personne atteinte de troubles moteurs d'origine neurologique utilisant un dispositif d'assistance selon un mode de réalisation possible de l'invention ;
- la figure 2 est un schéma illustrant les accélérations selon la direction verticale mesurées par un accéléromètre montrant un enrayage cinétique.

### DESCRIPTION DETAILLEE

On entend par verticale dans la présente description une direction parallèle à la direction de la pesanteur ou perpendiculaire au plan de l'horizon. On entend par horizontale une direction perpendiculaire à la verticale, donc perpendiculaire à la direction de la pesanteur.

On entend par posture la position du corps à un instant donné, par opposition à un mouvement. Une posture est par exemple la position debout ou la position assise.

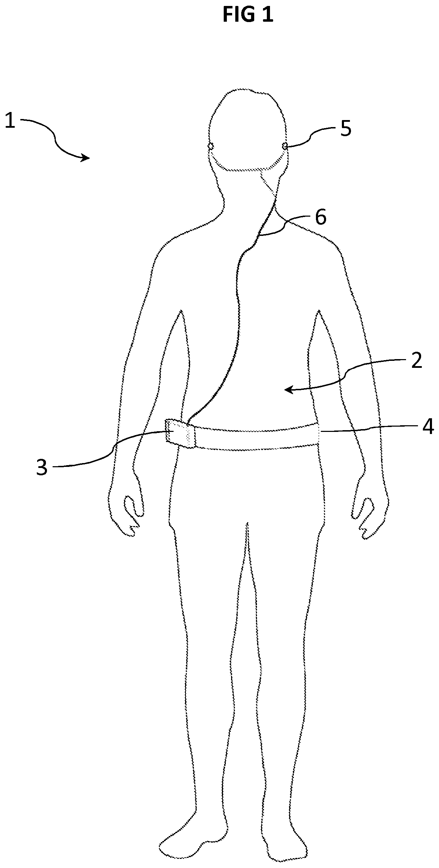

En référence à la figure 1, un dispositif d'assistance comprend un boîtier 3 dans lequel est logé un accéléromètre adapté à être porté par la personne 1 pour mesurer des accélérations selon au moins une direction. De préférence, l'accéléromètre est adapté pour être porté sur le tronc 2 de la personne. On entend par tronc la partie du corps humain allant du bassin aux épaules. L'accéléromètre est par exemple un accéléromètre piézoélectrique comprenant une structure de surface en polysilicium suspendu au-dessus d'une galette de silicium par un ressort en silicium présentant une résistance contre les forces d'accélération. La déformation de la structure est mesurée au moyen d'une capacité différentielle.

L'accéléromètre peut faire partie d'une centrale inertielle, comprenant, outre un accéléromètre capable de mesurer des accélérations dans une ou plusieurs directions, un ou plusieurs gyromètres capables de mesurer des vitesses angulaires et/ou un ou plusieurs gyroscopes capables de mesurer des positions angulaires. Ainsi, de préférence, le dispositif comprend une centrale inertielle de laquelle fait partie l'accéléromètre, ladite centrale inertielle étant configurée pour déterminer une orientation du tronc de la personne. Comme indiqué pour l'accéléromètre, la centrale inertielle est de préférence adaptée pour être portée sur le tronc de la personne.

L'accéléromètre est de préférence adapté pour être porté au niveau de la taille. Ainsi sur la figure 1, la personne atteinte de la maladie de parkinson porte autour de la taille une ceinture 4 munie du boîtier 3 comprenant ce premier accéléromètre. De préférence, l'accéléromètre est adapté pour mesurer des accélérations selon au moins une deuxième direction, différente de la première direction, et de préférence orthogonale à la première direction. Typiquement, l'accéléromètre est capable de déterminer des mesures d'accélération selon trois directions orthogonales entre elles, et donc de reconstituer l'accélération selon n'importe quelle direction, ainsi que l'orientation de l'accéléromètre, et donc du tronc 2 de la personne 1.

Ainsi, qu'elle soit directement mesurée ou reconstruite, la première direction correspond à la direction horizontale par rapport à la personne qui porte le dispositif. Plus précisément, la première direction est essentiellement horizontale, c'est-à-dire que la composante horizontale de la première direction est prépondérante sur la direction verticale.

Le capteur de mouvement comprenant l'accéléromètre est adapté pour être positionné sur l'utilisateur. L'accéléromètre peut notamment être positionné au niveau du tronc, des hanches, du plexus, ou des membres inférieurs de l'utilisateur. De préférence, le capteur de mouvement est positionné de sorte à pouvoir mesurer les accélérations au niveau de la hanche, du buste ou du plexus. Le capteur de mouvement est ainsi positionné au niveau d'une de ces parties du corps. En effet, la mesure des accélérations en ces endroits permet de prendre en compte les mouvements de l'ensemble des deux jambes, ce que ne permet pas un accéléromètre placé sur un pied, tandis qu'au-dessus du torse, par exemple aux épaules, l'amortissement par le corps est trop important. Ainsi, de préférence encore, le capteur de mouvement est positionné au niveau de la crête iliaque de la personne 1.

Le boîtier 3 comprend également des moyens de calcul, par exemple un processeur ou un ordinateur, un microcontrôleur, une unité de calcul, ou une unité arithmétique et logique, et des moyens de mémorisation. Les moyens de calcul sont connectés à l'accéléromètre, par exemple par une liaison sans fil ou par ondes ou par une liaison filaire, et aux moyens de mémorisation, par exemple par une liaison physique. Les moyens de calculs et les moyens de mémorisation sont typiquement disposés sur la même carte électronique, ou sur plusieurs cartes séparées et connectées entre elles.

Les moyens de mémorisation mémorisent au moins une pluralité de valeurs seuils d'accélération de prévention préalablement définies pour la direction horizontale. Ces valeurs seuils d'accélération de prévention sont associées à des postures de la personne, et des durées déterminées sont associées à chaque valeur seuil d'accélération d'anticipation. Plus précisément, une durée déterminée associée à une valeur seuil d'accélération d'anticipation est fonction du sens de franchissement de ladite valeur seuil d'accélération d'anticipation. Ainsi, pour chaque valeur seuil d'accélération d'anticipation, il y a une durée déterminée pour le franchissement depuis des accélérations inférieures à ladite valeur seuil vers des accélérations supérieures à ladite valeur seuil, et une durée déterminée pour le franchissement depuis des accélérations supérieures à ladite valeur seuil vers des accélérations inférieures à ladite valeur seuil, et ces deux durées déterminées sont différentes pour au moins la plupart des valeurs seuils.

Cependant, afin de mettre en œuvre différentes fonctionnalités du dispositif, les moyens de mémorisation peuvent également mémoriser d'autres valeurs, et notamment parmi elles au moins une valeur seuil d'accélération d'alerte, une valeur seuil de temps, une valeur seuil d'accélération de marche, et/ou une valeur seuil de rythme cardiaque.

Le dispositif contient également des moyens d'émission d'une stimulation auditive perceptible par la personne portant l'accéléromètre. Les moyens d'émission comprennent un transducteur 5 adapté pour émettre des sons, qui peut prendre la forme d'une oreillette à placer dans le canal auditif du porteur du dispositif, ou bien un transducteur adapté pour être placé contre un os temporal du porteur afin de stimuler ledit os, comme par exemple un appareil mettant en œuvre de l'ostéophonie. Les moyens d'émission sont reliés au boîtier 3, et plus précisément au moyen de calcul, par une liaison 6, qui peut être filaire comme sur la figure 1, ou bien être sans fil ou par ondes.

Le dispositif peut également comprendre un cardiofréquencemètre, adapté pour mesure le rythme cardiaque de la personne portant le dispositif. Celui-ci peut être porté lors de l'utilisation du dispositif pour l'assistance à la motricité, ou bien seulement lors du calibrage de celui-ci. Le dispositif comprend également au moins une batterie alimentant les différents composants du dispositif en énergie électrique.

Dans le dispositif d'assistance, les moyens de calcul sont configurés pour :
a) déterminer une posture de la personne à partir des accélérations mesurées,
b) comparer les accélérations mesurées par ledit accéléromètre dans la première direction par rapport à au moins une valeur seuil d'accélération d'anticipation associée à ladite posture ;
c) commander l'émission d'une stimulation auditive en fonction du franchissement de ladite valeur seuil d'accélération d'anticipation associée à ladite posture pendant une durée déterminée associée à ladite valeur seuil d'accélération d'anticipation.

Le dispositif est ainsi configuré pour stimuler le porteur dudit dispositif lorsqu'il est en mouvement pendant des phases de probabilité de survenue d'un phénomène d'enrayage cinétique de la marche, ces phases étant déterminées par la comparaison entre les accélérations mesurées et des valeurs seuil d'accélération d'anticipation. Plus précisément, il s'agit de comparer les amplitudes de ces accélérations avec des valeurs seuil d'accélération d'anticipation.

La probabilité de cette survenue dépend également de la posture de l'utilisateur. Ainsi, chaque valeur seuil d'accélération d'anticipation est associée à une posture. Plusieurs valeurs de seuil d'accélération peuvent être associées à une même posture. La direction prise en compte ici est celle de l'horizontale, et le sens des accélérations est celui du déplacement vers l'avant de l'utilisateur.

Afin de ne pas stimuler inutilement l'utilisateur, l'émission de la stimulation est conditionnée au franchissement d'une valeur seuil d'accélération d'anticipation pendant une durée déterminée qui lui est associée. Typiquement, la durée déterminée pendant laquelle l'accélération doit dépasser le seuil est comprise entre 150 ms et 2,5 secondes. La durée déterminée pendant laquelle l'accélération doit dépasser le seuil dépend de la valeur seuil d'accélération d'anticipation, qui elle-même dépend de la posture. Par conséquent, la durée déterminée dépend également de la posture. Les postures peuvent comprendre la posture assis-couché et la posture debout. A titre d'exemple, si la posture de l'utilisateur est déterminée comme étant assise ou couchée, la durée prédéterminée peut être comprise entre 200 ms et 1 seconde. Si la posture de l'utilisateur est déterminée comme étant debout, la durée prédéterminée peut être comprise entre 150 ms et 2,5 secondes. Cette durée déterminée peut dépendre du sens de franchissement, ainsi qu'expliqué plus haut.

La posture de la personne peut être déterminée à partir des accélérations mesurées. En complément, ou en alternative, elle peut également être déterminée à partir de l'orientation du tronc de l'utilisateur qu'une centrale inertielle, portée sur ce tronc, peut déterminer.

La mesure des accélérations permet de déterminer l'orientation du dispositif. On peut par exemple déterminer la pesanteur ou intégrer les accélérations. En fonction de l'orientation du dispositif, on peut déterminer la position de la personne, et donc sa posture en combinaison avec les accélérations. On peut donc déterminer la posture de la personne par les accélérations. Par exemple, on peut déterminer que la personne est assise ou couchée si le dispositif est incliné vers l'arrière, avec un dépassement d'un seuil d'inclinaison pendant une certaine durée (plusieurs secondes). De même, on peut également déterminer que la personne est debout si la position du dispositif est parallèle ou perpendiculaire au sol (en fonction des configurations), de façon stable (sur plusieurs secondes).

Les postures peuvent comprendre la posture assis-couché et la posture debout. Dans le cas de la posture assis-couché, une valeur seuil d'accélération d'anticipation associée à la posture assis-couché correspond à un déséquilibre vers l'arrière, la stimulation auditive étant émise lorsque les accélérations mesurées sont supérieures à ladite valeur seuil d'accélération d'anticipation associée à la posture assis-couché correspondant à un déséquilibre vers l'arrière. Par exemple, la valeur seuil d'accélération d'anticipation associée à la posture assis-couché correspondant à un déséquilibre vers l'arrière est entre - 1,2 m.s-² et -1,7 m.s-², par exemple de -1,5 m.s-², et la durée prédéterminée qui y est associée est comprise entre 0,5 et 1 seconde.

A titre d'exemple, si la posture de l'utilisateur est déterminée comme étant assise ou couchée, la stimulation est émise lorsque des accélérations supérieures à -1,5 m.s⁻² dans la direction horizontale sont mesurées pendant au moins 0,5 s. Autrement, aucune stimulation n'est émise.

Dans le cas de la posture debout, une valeur seuil d'accélération d'anticipation associée à la posture debout correspondant à un déséquilibre vers l'arrière et une valeur seuil d'accélération d'anticipation associée à la posture debout correspondant à un déplacement vers l'avant. La stimulation auditive est émise lorsque les accélérations mesurées sont inférieures à la valeur seuil d'accélération d'anticipation associée à la posture debout correspondant à un déséquilibre vers l'arrière et lorsque les accélérations mesurées sont supérieures à la valeur seuil d'accélération d'anticipation associée à la posture debout correspondant à un mouvement vers l'avant.

Par exemple, la valeur seuil d'accélération d'anticipation associée à la posture debout correspondant à un déséquilibre vers l'arrière est entre -0,8 m.s-² et -1,2 m.s-², par exemple de -1 m.s-², et la durée prédéterminée qui y est associée est entre 150 et 250 ms, par exemple de 200 ms, et la valeur seuil d'accélération d'anticipation associée à la posture debout correspondant à un déplacement vers l'avant est entre 2,5 et 3 m.s-², par exemple de 2,8 m.s⁻², et la durée prédéterminée qui y est associée est entre 150 et 250 ms, par exemple de 200 ms.

A titre d'exemple, si la posture de l'utilisateur est déterminée comme étant debout, la stimulation est émise lorsque sont mesurées des accélérations supérieures à 2,8 m.s⁻² pendant 0,2 s ou entre -1,5 m.s⁻² et -1 m.s⁻² pendant 0,2 s. Autrement, aucune stimulation n'est émise.

Le tableau 1 ci-dessous permet de donner un exemple de seuils utilisés, en fonction de la posture déterminée par le dispositif.

| Posture | Accélérations sur l'axe horizontal (en m.s⁻²) | | | |
|---|---|---|---|---|
| | -infini -1,5 -1 2,8 +infini | | | |
| Assis-couché | Pas de stimulation | stimulation | Pas de stimulation | Pas de stimulation |
| Debout | Pas de stimulation | stimulation | Pas de stimulation | stimulation |

Le tableau 2 ci-dessous donne des exemples de durée prédéterminées qui peuvent être associées aux valeurs seuils d'accélérations données dans le tableau 1, en fonction du sens de franchissement, pour différentes postures déterminées par le dispositif.

| Posture | Accélérations | Franchissement sens positif | Franchissement sens négatif |
|---|---|---|---|
| Assis-couché | -1,5 | 350 ms | 500 ms |
| Debout | -1 | 2500 ms | 200 ms |
| | 2,8 | Entre 200 et 500 ms | Entre 350 et 2500 ms |

On appelle ici "franchissement sens positif" un franchissement du seuil depuis des accélérations inférieures à ladite valeur seuil vers des accélérations supérieures à ladite valeur seuil, et "franchissement sens négatif" un franchissement depuis des accélérations supérieures à ladite valeur seuil vers des accélérations inférieures à ladite valeur seuil.

Lorsque les conditions de stimulation sont réunies, les moyens de calcul commandent aux moyens d'émission d'émettre la stimulation auditive selon une période de référence correspondant à une fréquence calculée des pas de la personne. La stimulation auditive comprend une pulsation rythmique, qui peut être isolée et correspondre par exemple à une répétition d'un court son, ou bien encore être couplée à d'autres sons, comme dans le cas d'une mélodie. Dans tous les cas, la pulsation rythmique est aisément identifiable par l'utilisateur. La période de la pulsation de la stimulation auditive est basée sur une période de référence mémorisée dans les moyens de mémorisation. La période de la pulsation est calculée en fonction de la période moyenne des pas lorsque la personne marche sans présenter d'enrayage cinétique. Elle varie donc en fonction des individus. A titre d'exemple, lorsque la personne marche avec une moyenne de 140 pas par minutes, cela revient à une période d'environ 0,43 seconde entre chaque pas. La période de la pulsation de la stimulation auditive est alors de 0,43 seconde.

La stimulation auditive peut être émise, selon les cas, tant que les conditions d'émission de celle-ci sont remplies, être poursuivie un certain temps même après que ces conditions aient cessé d'être remplies et/ou ou s'arrêter après une durée déterminée même lorsque les conditions sont remplies. Par exemple, dans le cadre du déséquilibre arrière, la stimulation auditive peut être émise tant que les accélérations mesurées sont comprises entre -1,5 m.s⁻² et -1 m.s⁻². Dans ce cas du déplacement vers l'avant, la stimulation peut être poursuivie pendant 40 à 50 secondes lorsque le seuil d'accélération correspondant est franchi (par exemple le seuil de 2,8 m.s⁻².

De préférence, les moyens de mémorisation mémorisent au moins une valeur seuil d'accélération d'alerte, et l'accéléromètre est également adapté pour mesurer des accélérations selon une seconde direction, ladite seconde direction correspondant à la verticale.

Dans le dispositif d'assistance, les moyens de calcul sont alors en outre configurés pour réaliser les étapes suivantes :
a) comparer les accélérations mesurées dans la seconde direction par rapport à la valeur seuil d'accélération d'alerte ;
b) générer un signal d'alerte lorsqu'une accélération mesurée a une valeur supérieure à la valeur seuil d'accélération d'alerte.

En effet, au début d'un enrayage cinétique, la personne atteinte de la maladie de Parkinson présente une marche disharmonieuse et asymétrique du point de vue anatomofonctionnel. Les pas deviennent moins amples, plus rapprochés, et plus rapides. Cela se traduit, en termes d'accélération verticales mesurées, par des amplitudes d'accélération plus importantes, qui vont dès lors dépasser une valeur seuil d'accélération d'alerte, et donc enclencher la génération d'un signal d'alerte. La valeur seuil d'accélération d'alerte est paramétrée en fonction de chaque personne, mais est typiquement comprise, en valeur absolue, entre 1 et 7 m.s⁻², plus précisément entre 2 et 6 m.s⁻², et encore plus précisément entre 3 et 5 m.s⁻².On peut ainsi détecter au plus vite la survenue d'un épisode d'enrayage cinétique. Ensuite, les moyens de calcul sont configurés pour commander aux moyens d'émission d'émettre une stimulation auditive en fonction du signal d'alerte.

Cependant, le déclenchement d'un signal d'alerte à chaque franchissement d'une valeur seuil d'accélération d'alerte entraîne de nombreux déclenchements intempestifs. En effet, une accélération forte peut être causée non seulement par un enrayage cinétique, mais également par d'autres facteurs, par exemple lorsque le pied heurte un obstacle.

Ainsi, de préférence, les moyens de calcul sont configurés pour commander aux moyens d'émission d'émettre une stimulation auditive à partir du moment où au moins deux signaux d'alerte sont générés dans un intervalle de temps inférieur à 0,5 seconde, de préférence inférieur à 0,3 seconde. Par conséquent, un dépassement isolé de la valeur seuil d'accélération d'alerte ne résulte pas dans la génération d'une stimulation auditive.

Néanmoins, même avec l'exigence de la proximité temporelle de deux dépassements de seuil, il peut y avoir de fausses alertes résultant en un déclenchement intempestif de stimulation auditive. On utilise donc la détection d'une fréquence rapprochée des pas. Pour ce faire, les moyens de mémorisation mémorisent une valeur seuil de temps et une valeur seuil d'accélération de marche, et les moyens de calculs sont configurés pour mesurer un intervalle de temps entre deux mesures d'accélérations dépassant ladite valeur seuil d'accélération de marche et déclencher une alerte lorsqu'un intervalle de temps entre deux dépassements consécutifs de ladite valeur seuil d'accélération de marche est inférieur à la valeur seuil de temps mémorisée.

La valeur seuil d'accélération de marche est inférieure à la valeur seuil d'accélération d'alerte. Par exemple, elle est inférieure, en valeur absolue, à 3m.s⁻². Le dépassement en amplitude de la valeur seuil d'accélération de marche par les accélérations mesurées correspond à la détection d'un pas. L'intervalle de temps entre deux dépassements consécutifs correspond donc à l'intervalle de temps entre deux pas. Lors d'un épisode d'enrayage cinétique, l'intervalle de temps entre les pas diminue. On adjoint donc au premier critère de dépassement du seuil d'accélération d'alerte un second critère portant sur la fréquence des pas. Cela permet de mieux détecter les épisodes correspondant réellement à un enrayage cinétique.

Par ailleurs, de fortes accélérations peuvent être détectées en l'absence d'enrayage cinétique, notamment lorsque la personne change de position, par exemple lorsqu'elle monte un escalier. Il convient dans ce cas de détecter cette situation ne correspondant pas à la marche. A cet effet, l'accéléromètre est adapté pour mesurer une accélération selon une autre direction différente de la deuxième direction, et de préférence perpendiculaire à celle-ci; et les moyens de mémorisation mémorisent une seconde valeur seuil d'accélération. Les moyens de calcul peuvent alors être configurés pour inhiber la génération d'un signal d'alerte pendant une durée prédéterminée lorsqu'une accélération mesurée à une valeur supérieure à cette seconde valeur seuil d'accélération d'alerte.

Plus généralement, les moyens de calcul sont configurés pour détecter à partir des mesures de l'accéléromètre, et/ou éventuellement par celles d'un gyromètre ou gyroscope, la position du corps et la situation de déplacement ou de repos de la personne portant ledit accéléromètre. La position du corps peut être déterminée au moyen de la détection de l'accélération de la pesanteur terrestre, qui indique la verticale, ou bien directement par le gyroscope. Les moyens de mémorisation mémorisent une valeur seuil d'accélération marche, et les moyens de calcul détectent une position de repos de la personne portant les accéléromètres lorsque toutes les valeurs mesurées par les accéléromètres sont inférieures à la valeur seuil de marche pendant une durée supérieure à un temps prédéterminé.

Ainsi, lorsque la personne est détectée comme étant debout, elle est considérée en repos quand seules sont mesurées des accélérations comprises dans un intervalle d'accélération, tel que -1 m.s⁻² et 2,8 m.s⁻² dans la direction verticale, pendant un intervalle de temps tel que 2,5 s. Lorsque la personne est détectée comme étant assise ou couchée, elle est considérée en repos quand seules sont mesurées des accélérations comprises dans un intervalle d'accélération, tel qu'entre -1,5 m.s⁻² et 1,5 m.s⁻² dans la direction verticale, pendant un intervalle de temps tel que 0,35 s.

Les moyens de calcul sont configurés pour comparer les accélérations mesurées par l'accéléromètre de façon périodique, la valeur de cette période étant égale à :
- une première période de mesure lorsque le tronc de la personne est non-vertical, c'est-à-dire lorsqu'elle est couchée ; et/ou
- une deuxième période de mesure inférieure à la première valeur, lorsque le tronc de la personne est vertical, c'est-à-dire lorsqu'elle est assise ; et
- une troisième période de mesure inférieure à la deuxième valeur, lorsque la personne marche.

Par exemple, la première période de mesure est supérieure à 75 ms, et est par exemple de 100 ms, tandis que la deuxième période de mesure est comprise entre 75 ms et 25 ms, et est par exemple de 50 ms, et la troisième période de mesure est inférieure à 25 ms, et est par exemple de 10 ms.

La stimulation auditive émise lors de la détection de la survenue d'un épisode d'enrayage cinétique est de préférence la même qu'en prévention de l'enrayage cinétique, et la période de la pulsation de la stimulation auditive est basée sur une période de référence mémorisée dans les moyens de mémorisation, liée à la valeur de temps utilisée pour détecter une fréquence trop rapide des pas. La période de la pulsation est calculée en fonction de la période moyenne des pas lorsque la personne marche sans présenter d'enrayage cinétique.

La stimulation auditive peut être émise dès la détection de l'enrayage cinétique, à l'aide des signaux d'alerte décrits précédemment, ou être émise à l'expiration d'une temporisation qui fait suite à la détection de l'enrayage cinétique. Cette temporisation peut être comprise de 0,1 à 37 secondes par exemple, mais, lorsqu'elle est présente, elle peut être d'au moins 2 secondes, par exemple de 10 secondes. Préalablement à la temporisation, un signal d'arrêt est émis par les moyens d'émission, indiquant à la personne de s'arrêter. Cela permet de ramener la personne à une motricité qu'elle contrôle, c'est-à-dire l'immobilité, puis de la faire repartir grâce à la stimulation auditive à la suite de la temporisation.

Cependant, l'arrêt de la personne peut entraîner des chutes, car elle peut se trouver en déséquilibre lors de l'enrayage cinétique. Par ailleurs, la durée de la temporisation ralentit d'autant les déplacements de la personne. Ainsi, la stimulation auditive est de préférence envoyée sans temporisation, où avec une temporisation très faible, inférieure à la dixième de seconde.

Par ailleurs, il est courant lors d'un enrayage cinétique que le rythme cardiaque de la personne augmente, et devienne subitement supérieur à un rythme cardiaque normal de marche de cette personne. Ce rythme cardiaque supérieur peut être utilisé pour distinguer un enrayage cinétique parmi d'autres cas pouvant éventuellement entraîner des accélérations similaires. A cet effet, le dispositif peut comprendre des moyens de mesure du rythme cardiaque adaptés à être portés par la personne pour mesurer son rythme cardiaque. Les moyens de mémorisation mémorisent alors une valeur seuil de rythme cardiaque et les moyens de calculs sont configurés pour générer un signal d'alerte lorsque le rythme cardiaque mesuré a une valeur supérieure à la valeur seuil de rythme cardiaque mémorisée, celle-ci correspondant à un rythme cardiaque normal de marche de cette personne, préalablement déterminé.

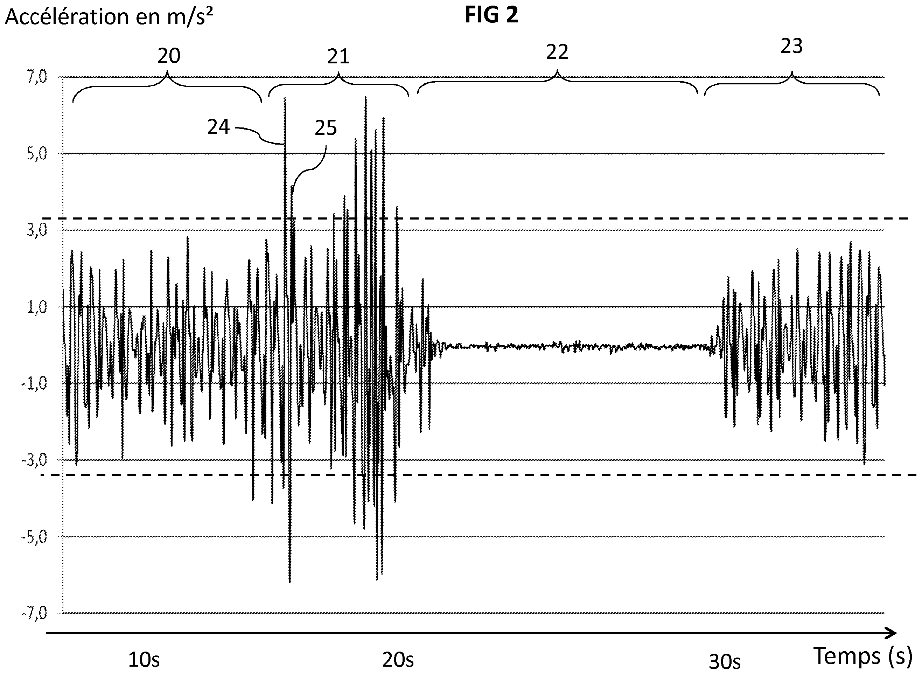

La figure 2 illustre les accélérations mesurées dans la direction verticale pour une personne atteinte de la maladie de parkinson lorsqu'elle marche et qu'elle subit un enrayage cinétique.

Dans une première partie 20, la personne marche sans présenter d'enrayage cinétique. Les accélérations mesurées sont en-deçà du seuil d'accélération d'alerte représenté par les lignes horizontales en tirets, en haut pour les valeurs positives et en bas pour les négatives. En outre, les pics d'accélération qui dépassent la valeur seuil d'accélération de marche sont espacés d'un intervalle de temps supérieur à la valeur seuil de temps.

Un calibrage du dispositif d'assistance est mis en œuvre pour adapter à la personne portant le dispositif les différentes valeurs utilisées dans le cadre de l'utilisation du dispositif, telle que la valeur seuil d'accélération d'alerte.

Dans une deuxième partie 21, un enrayage cinétique se produit. Les amplitudes des accélérations sont plus importantes, et dépassent la valeur seuil d'accélération d'alerte. En outre, les pics d'accélération qui dépassent la valeur seuil d'accélération de marche se rapprochent : les intervalles de temps les espaçant sont inférieurs à la valeur seuil de temps.

Les deux premiers dépassements 24, 25 de la valeur seuil d'accélération d'alerte se produisent dans un intervalle de temps inférieur à quelques dixièmes de secondes. Les moyens de calcul commandent alors aux moyens d'émission d'émettre la stimulation auditive après une temporisation de 10 secondes. Pendant cette temporisation de 10 secondes, la personne subissant l'enrayage cinétique est à l'arrêt, ce qui correspond aux valeurs quasi-nulles d'accélération de la partie 22. Un son stop peut être émis pour indiquer à la personne de s'arrêter, ce qui lui évite de tomber. Il est également possible d'émettre la stimulation auditive dès la détection de l'enrayage cinétique, sans temporisation.

Ici, le signal audible est émis à partir 30^{ème} seconde. La quatrième partie 23 correspond ainsi à la reprise de la marche par la personne entendant la stimulation auditive. On constate que les pics d'accélérations sont régulièrement espacés avec des amplitudes régulières, indiquant ainsi la reprise d'une marche régulière et harmonieuse.

Dans le cadre de ce procédé de calibration, l'accéléromètre est disposé sur la personne, notamment au niveau de sa ceinture, c'est-à-dire au niveau de la crête iliaque, comme illustré sur la figure 1. Comme précédemment, l'accéléromètre peut être disposé au niveau du tronc, des hanches, du plexus, ou membres inférieurs. La personne marche ensuite, et au moins une série de mesures d'accélération est acquise pendant la marche de la personne, au moyen de l'accéléromètre.

Ces mesures d'accélérations sont utilisées pour déterminer les paramètres (amplitude des accélérations, rythme ...) d'une marche normale de la personne, c'est-à-dire en l'absence d'épisode d'enrayage cinétique. À cet effet, la série de mesures d'accélération est amputée des parties correspondant à une motricité altérée de la personne, c'est-à-dire aux parties correspondant à des épisodes d'enrayage cinétique, et plus généralement à une marche non normale. Cette sélection dans la série de mesures d'accélération peut être faite manuellement par un professionnel qui observe la marche du patient, dans la mesure où un épisode d'enrayage cinétique est clairement identifiable par un professionnel. La sélection peut aussi être basée sur l'observation de des mesures, puisque, comme sur la figure 2, les parties correspondant à une marche normale et les parties correspondant à un enrayage cinétique ou à un arrêt sont facilement identifiables.

Ensuite, à partir de la série ainsi amputée, il est possible de déterminer les paramètres d'une marche normale. En particulier, un calcul sur les données des accélérations permet de déterminer le seuil d'accélération d'alerte au-delà duquel l'accélération mesurée est considérée comme anormale et pouvant indiquer la survenue d'un enrayage cinétique. Le calcul sur les données des accélérations pour déterminer le seuil d'accélération d'alerte peut par exemple faire intervenir des notions statistiques telles que la moyenne des amplitudes des accélérations et leur écart-type.

Le seuil d'accélération d'alerte peut être évolutif, avec une première détermination au moyen d'une première série d'échantillons extraits de la série amputée, puis une confirmation ou une modification de ce seuil au moyen d'autres séries d'échantillons, extraits de la même série amputée ou d'une autre série amputée issu de la même personne.

La valeur seuil d'accélération de marche peut également être déterminée à partir la moyenne des accélérations. La valeur seuil de temps peut également être déterminée à partir de la série amputée, en détectant les pics d'accélération correspondant à un pas et en déterminant ainsi le rythme de la marche.

Par ailleurs, lors de la marche pendant laquelle est relevée la série d'accélérations, la personne porte de préférence un cardiofréquencemètre, et son rythme cardiaque est mesuré en même temps que l'accélération. Ce rythme cardiaque peut être utilisé pour ne conserver que les parties de la série correspondant à une marche normale : les parties associées à un rythme cardiaque plus élevé que la moyenne lors de sa marche sont écartés.

En effet, ces parties peuvent indiquer non seulement la survenue d'un enrayage cinétique, mais encore d'autres évènements de stress qui pourraient résulter en une marche anormale.

Par ailleurs, que ce soit pour la mise en œuvre du procédé de calibration ou pour un suivi de la personne portant le dispositif lors de son utilisation, les moyens de calculs sont de préférence configurés pour mémoriser, sur les moyens de mémorisation, les mesures réalisées par au moins par au moins un dispositif de mesure du dispositif d'assistance, tel que l'accéléromètre et/ou le cardiofréquencemètre. Il est alors possible de procéder à des recalibrages périodiques du dispositif à partir des mesures relevées par celui-ci lors de l'utilisation du dispositif, et stockées dans les moyens de mémorisation. On peut ainsi prévoir une périodicité de recalibrage comprise entre plusieurs fois par jour et une fois par semaine.

L'invention n'est pas limitée au mode de réalisation décrit et représenté aux figures annexées. Des modifications restent possibles, notamment du point de vue de la constitution des divers éléments, sans sortir pour autant du domaine de protection de l'invention défini par les revendications jointes.

## Revendications

1. Dispositif d'assistance destiné à une personne (1) atteinte de troubles moteurs d'origine neurologique susceptibles de provoquer un enrayage cinétique, comprenant :
• un accéléromètre adapté pour être porté par la personne (1) pour mesurer des accélérations selon au moins une première direction qui correspond à l'axe horizontal ; et
• des moyens (5) d'émission d'une stimulation auditive perceptible par la personne portant l'accéléromètre; et
• des moyens de calcul connectés à l'accéléromètre, à des moyens de mémorisation et aux moyens d'émission ;
les moyens de mémorisation mémorisant une pluralité de valeurs seuils d'accélération d'anticipation préalablement définies pour ladite première direction, chaque valeur seuil d'accélération d'anticipation étant associée à une posture, et les moyens de calcul étant configurés pour :
a) déterminer une posture de la personne à partir des accélérations mesurées,
b) comparer les accélérations mesurées par ledit accéléromètre dans la première direction par rapport à au moins une valeur seuil d'accélération d'anticipation associée à ladite posture;
c) commander l'émission d'une stimulation auditive en fonction du franchissement de ladite valeur seuil d'accélération d'anticipation associée à ladite posture;
le dispositif d'assistance étant **caractérisé en ce que** les moyens de calcul sont configurés pour :
- associer également chaque posture à une durée déterminée fonction du sens de franchissement de ladite valeur seuil d'accélération d'anticipation par les accélérations mesurées, et
- commander l'émission de la stimulation auditive en fonction du franchissement de ladite valeur seuil d'accélération d'anticipation associée à ladite posture pendant la durée déterminée.

2. Dispositif selon la revendication précédente, dans lequel la posture est une posture assis-couché, et une valeur seuil d'accélération d'anticipation associée à la posture assis-couché correspond à un déséquilibre vers l'arrière, les moyens de calcul étant configurés commander l'émission de la stimulation auditive lorsque les accélérations mesurées sont supérieures à ladite valeur seuil d'accélération d'anticipation associée à la posture assis-couché correspondant à un déséquilibre vers l'arrière.

3. Dispositif selon l'une des revendications précédentes, dans lequel la posture est une posture debout, une valeur seuil d'accélération d'anticipation associée à la posture debout correspondant à un déséquilibre vers l'arrière et une valeur seuil d'accélération d'anticipation associée à la posture debout correspondant à un déplacement vers l'avant, les moyens de calcul étant configurés commander l'émission de la stimulation auditive lorsque les accélérations mesurées sont inférieure à la valeur seuil d'accélération d'anticipation associée à la posture debout correspondant à un déséquilibre vers l'arrière et lorsque les accélérations mesurées sont supérieures à la valeur seuil d'accélération d'anticipation associée à la posture debout correspondant à un mouvement vers l'avant.

4. Dispositif selon l'une des revendications précédentes, comprenant une centrale inertielle de laquelle fait partie l'accéléromètre, ladite centrale inertielle étant adaptée pour être portée sur le tronc de la personne et étant en outre configurée pour déterminer une orientation du tronc de la personne, ladite orientation étant utilisée pour déterminer la posture de la personne.

5. Dispositif d'assistance selon l'une des revendications précédentes, dans lequel les moyens de calcul sont configurés pour commander aux moyens d'émission d'émettre la stimulation auditive selon une période de référence correspondant à une fréquence calculée des pas de la personne.

6. Dispositif selon l'une des revendications précédentes, dans lequel l'accéléromètre est également adapté pour mesurer des accélérations selon une seconde direction, ladite seconde direction correspondant à la verticale, et les moyens de mémorisation mémorisent au moins une valeur seuil d'accélération d'alerte préalablement définie dans une seconde direction, les moyens de calculs étant en outre configurés pour
a) comparer les accélérations mesurées dans la seconde direction par rapport à ladite au moins une valeur seuil d'accélération d'alerte,
b) générer un signal d'alerte lorsqu'une accélération mesurée a une valeur supérieure à la valeur seuil d'accélération d'alerte ;
c) commander aux moyens d'émission d'émettre une stimulation auditive en fonction du signal d'alerte.

7. Dispositif selon la revendication précédente, **caractérisé en ce que** les moyens de mémorisation mémorisent une valeur seuil de temps et une valeur seuil d'accélération de marche, et **en ce que** les moyens de calculs sont configurés pour mesurer un intervalle de temps entre deux mesures d'accélérations dépassant ladite valeur seuil d'accélération de marche et déclencher une alerte lorsqu'un intervalle de temps entre deux dépassements consécutifs de ladite valeur seuil d'accélération de marche est inférieur à la valeur seuil de temps mémorisée.

8. Dispositif d'assistance selon l'une des revendications 6 à 7, dans lequel les moyens de mémorisation mémorisent une période de référence correspondant à la valeur seuil de temps, et les moyens de calcul sont configurés pour commander aux moyens d'émission d'émettre la stimulation auditive selon la période de référence.

9. Dispositif d'assistance selon l'une des revendications 6 à 7, **caractérisé en ce que** les moyens de calcul sont configurés pour commander aux moyens d'émission d'émettre une stimulation auditive à partir du moment où au moins deux signaux d'alerte sont générés dans un intervalle de temps inférieur à 0,5 seconde, de préférence inférieur à 0,3 seconde.

10. Dispositif d'assistance selon l'une des revendications précédentes, **caractérisé en ce qu'**il comprend :
- des moyens de mesure du rythme cardiaque adaptés à être portés par la personne pour mesurer son rythme cardiaque ; et **en ce que**
- les moyens de mémorisation mémorisent une valeur seuil de rythme cardiaque ;
et **en ce que** les moyens de calculs sont configurés pour générer un signal d'alerte lorsque le rythme cardiaque mesuré a une valeur supérieure à la valeur seuil de rythme cardiaque mémorisée.

11. Dispositif d'assistance selon l'une des revendications dépendantes, dans lequel l'accéléromètre est configuré pour mesurer une accélération selon une autre direction différente de celle de la seconde direction, et les moyens de mémorisation mémorisent une seconde valeur seuil d'accélération d'alerte associée à ladite autre direction, et les moyens de calculs sont configurés pour inhiber la génération d'un signal d'alerte pendant une durée prédéterminée lorsqu'une accélération mesurée dans ladite autre direction a une valeur supérieure à la seconde valeur seuil d'accélération mémorisée.

12. Dispositif d'assistance selon la revendication précédente, dans lequel les moyens de mémorisation mémorisent une valeur seuil d'accélération marche et une valeur de temps repos, et les moyens de calcul sont configurés pour détecter une position assise ou couchée de la personne portant l'accéléromètre lorsque toutes les valeurs d'accélération mesurées par l'accéléromètre sont inférieures à la valeur seuil d'accélération marche pendant une durée supérieure à la valeur de temps repos.

13. Dispositif d'assistance selon l'une des deux revendications précédentes, **caractérisé en ce que** les moyens de calcul sont configurés pour comparer les accélérations mesurées par les accéléromètres de façon périodique, la valeur de cette période étant égale à :
- une première période de mesure lorsque le tronc de la personne est non-vertical ; et/ou
- une deuxième période de mesure inférieure à la première valeur, lorsque le tronc de la personne est vertical ; et
- une troisième période de mesure inférieure à la deuxième valeur, lorsque la personne marche.

14. Procédé de calibration d'un dispositif d'assistance selon l'une des revendications précédentes, comprenant les étapes selon lesquelles :
- l'accéléromètre est disposé sur le tronc de la personne,
- une série de mesures d'accélération est acquise pendant que la personne marche,
- la série est amputée des parties de la série de mesures d'accélération correspondant à une motricité altérée de la personne,
- les valeurs de seuils d'accélération d'anticipation et/ou la valeur seuil d'accélération d'alerte sont déterminées à partir des accélérations de la série amputée.

15. Procédé de calibration selon la revendication précédente, dans lequel la valeur seuil d'accélération de marche et/ou la valeur seuil de temps est déterminée à partir de la moyenne des accélérations de la série amputée.

## Patentansprüche

1. Hilfsvorrichtung für eine Person (1) mit motorischen Störungen neurologischen Ursprungs, die geeignet sind, eine kinetische Hemmung hervorzurufen, umfassend:
• einen Beschleunigungsmesser, der geeignet ist, von der Person (1) getragen zu sein, um Beschleunigungen gemäß wenigstens einer ersten Richtung zu messen, die der horizontalen Achse entspricht; und
• Sendemittel (5) einer auditiven Stimulation, die von der den Beschleunigungsmesser tragenden Person wahrnehmbar ist; und
Berechnungsmittel, die an den Beschleunigungsmesser, Speichermittel und Sendemittel angeschlossen sind;
Speichermittel, die eine Vielzahl von Grenzwerten einer erwarteten Beschleunigung speichern, die zuvor für die genannte erste Richtung festgelegt worden waren, wobei jeder Grenzwert einer erwarteten Beschleunigung einer Haltung zugeordnet ist, und die Berechnungsmittel ausgestaltet sind zum:
a) Bestimmen einer Haltung der Person ausgehend von gemessenen Beschleunigungen,
b) Vergleichen der Beschleunigungen, die von dem genannten Beschleunigungsmesser in der ersten Richtung gemessen sind, in Bezug auf wenigstens einen Grenzwert einer erwarteten Beschleunigung, der der genannten Haltung zugeordnet ist;
c) Befehlen des Sendens einer auditiven Stimulation in Abhängigkeit von der Überschreitung des genannten Grenzwertes einer erwarteten Beschleunigung, die der genannten Haltung zugeordnet ist;
wobei die Hilfsvorrichtung **dadurch gekennzeichnet ist, dass** die Berechnungsmittel ausgestaltet sind um:
- jeder Haltung ebenfalls eine Dauer zuzuordnen, die in Abhängigkeit von der Überschreitungsrichtung des genannten Grenzwertes einer erwarteten Beschleunigung von den gemessenen Beschleunigungen bestimmt ist, und
- das Senden der auditiven Stimulation in Abhängigkeit von der Überschreitung des genannten Grenzwertes einer erwarteten Beschleunigung zu befehlen, die der genannten Haltung während der bestimmten Dauer zugeordnet ist.

2. Vorrichtung gemäß dem voranstehenden Anspruch, bei der die Haltung eine sitzende, liegende Haltung ist, und ein Grenzwert einer erwarteten Beschleunigung, die der sitzenden, liegenden Haltung zugeordnet ist, einem Ungleichgewicht nach hinten entspricht, wobei die Berechnungsmittel zum Befehlen des Sendens der auditiven Stimulation ausgestaltet sind, wenn die gemessenen Beschleunigungen größer sind als der genannte Grenzwert einer erwarteten Beschleunigung, die der sitzenden, liegenden Haltung zugeordnet ist, die einem Ungleichgewicht nach hinten entspricht.

3. Vorrichtung gemäß einem der voranstehenden Ansprüche, bei der die Haltung eine stehende Haltung ist, wobei ein Grenzwert einer erwarteten Beschleunigung, die der stehenden Haltung zugeordnet ist, einem Ungleichgewicht nach hinten entspricht, und ein Grenzwert einer erwarteten Beschleunigung, die der stehenden Haltung zugeordnet ist, einer Verschiebung nach vorne entspricht, wobei die Berechnungsmittel zum Befehlen des Sendens der auditiven Stimulation ausgestaltet sind, wenn die gemessenen Beschleunigungen geringer sind als der Grenzwert einer erwarteten Beschleunigung, die der stehenden Haltung zugeordnet ist, die einem Ungleichgewicht nach hinten entspricht und wenn die gemessenen Beschleunigungen größer sind als der Grenzwert der erwarteten Beschleunigung, die der stehenden Haltung zugeordnet ist, die einer Bewegung nach vorn entspricht.

4. Vorrichtung gemäß einem der voranstehenden Ansprüche, umfassend eine Trägheitszentrale, zu der der Beschleunigungsmesser gehört, wobei die genannte Trägheitszentrale geeignet ist, um auf dem Rumpf der Person getragen zu sein und darüber hinaus ausgestaltet ist, um eine Ausrichtung des Rumpfes der Person zu bestimmen, wobei die genannte Ausrichtung zum Bestimmen der Haltung der Person verwendet ist.

5. Hilfsvorrichtung gemäß einem der voranstehenden Ansprüche, bei der die Berechnungsmittel ausgestaltet sind, um den Sendemitteln das Senden der auditiven Stimulation gemäß einer Referenzperiode zu befehlen, die einer berechneten Frequenz der Schritte der Person entspricht.

6. Vorrichtung gemäß einem der voranstehenden Ansprüche, bei der der Beschleunigungsmesser ebenfalls geeignet ist, um Beschleunigungen gemäß einer zweiten Richtung zu messen, wobei die genannte zweite Richtung der Vertikalen entspricht, und wobei die Speichermittel wenigstens einen Warn-Grenzwert einer Beschleunigung speichern, die zuvor in einer zweiten Richtung definiert ist, wobei die Berechnungsmittel darüber hinaus ausgestaltet sind zum/um
a) Vergleichen der Beschleunigungen, die in der zweiten Richtung gemessen sind, in Bezug auf wenigstens einen Warn-Grenzwert einer Beschleunigung;
b) Erzeugen eines Warnsignals, wenn eine gemessene Beschleunigung einen höheren Wert als den Warn-Grenzwert einer Beschleunigung aufweist;
c) den Sendemitteln in Abhängigkeit des Warnsignals eine auditive Stimulation zu befehlen.

7. Vorrichtung gemäß dem voranstehenden Anspruch, **dadurch gekennzeichnet, dass** die Speichermittel einen Zeit-Grenzwert und einen Gang-Grenzwert der Beschleunigung speichern und dass die Berechnungsmittel zum Messen eines Zeitintervalls zwischen zwei Beschleunigungsmessungen ausgestaltet sind, die den genannten Gang-Grenzwert der Beschleunigung überschreiten und eine Warnung auslösen, wenn ein Zeitintervall zwischen zwei aufeinander folgenden Überschreitungen des genannten Gang-Grenzwerts der Beschleunigung niedriger ist als der gespeicherte Zeit-Grenzwert.

8. Hilfsvorrichtung gemäß einem der Ansprüche 6 bis 7, bei der die Speichermittel eine Referenzperiode speichern, die dem Zeit-Grenzwert entspricht, und die Berechnungsmittel ausgestaltet sind, um den Sendemittel das Senden der auditiven Stimulation gemäß der Referenzperiode zu befehlen.

9. Hilfsvorrichtung gemäß einem der Ansprüche 6 bis 7, **dadurch gekennzeichnet, dass** die Berechnungsmittel ausgestaltet sind, um den Sendemitteln das Senden einer auditiven Stimulation ausgehend von dem Moment zu befehlen, an dem wenigstens zwei Warnsignale in einem Zeitintervall von weniger als 0,5 Sekunden, bevorzugt weniger als 0,3 Sekunden erzeugt sind.

10. Hilfsvorrichtung gemäß einem der voranstehenden Ansprüche, **dadurch gekennzeichnet, dass** sie umfasst:
- Messmittel des Herzrhythmus, die geeignet sind, um von der Person getragen zu werden, um ihren Herzrhythmus zu messen; und dass
- die Speichermittel einen Grenzwert des Herzrhythmus speichern;
und dass die Berechnungsmittel ausgestaltet sind, um ein Warnsignal zu erzeugen, wenn der gemessene Herzrhythmus einen Wert aufweist, der größer ist als der gespeicherte Grenzwert des Herzrhythmus.

11. Hilfsvorrichtung gemäß einem der abhängigen Ansprüche, bei der der Beschleunigungsmesser zum Messen einer Beschleunigung gemäß einer anderen Richtung als der ausgestaltet ist, die von der der zweiten Richtung unterschiedlich ist, und die Speichermittel einen zweiten Warn-Grenzwert einer Beschleunigung speichern, die der genannten anderen Richtung zugeordnet ist, und die Berechnungsmittel ausgestaltet sind, um das Erzeugen eines Warnsignals während einer vorbestimmten Dauer zu hemmen, wenn eine gemessene Beschleunigung in der genannten anderen Richtung einen größeren Wert als den zweiten gespeicherten Beschleunigungs-Grenzwert aufweist.

12. Hilfsvorrichtung gemäß dem voranstehenden Anspruch, bei der die Speichermittel einen Gang-Grenzwert der Beschleunigung und einen Ruhezeit-Wert speichern und die Berechnungsmittel ausgestaltet sind, um eine sitzende oder liegende Position der den Beschleunigungsmesser tragenden Person zu detektieren, wenn alle von dem Beschleunigungsmesser gemessenen Beschleunigungswerte niedriger sind als der Gang-Grenzwert der Beschleunigung während einer Dauer, die den Ruhezeitwert übersteigt.

13. Hilfsvorrichtung gemäß einem der voranstehenden Ansprüche, **dadurch gekennzeichnet, dass** die Berechnungsmittel zum Vergleichen der von den Beschleunigungsmessern periodisch gemessenen Beschleunigungen ausgestaltet sind, wobei der Wert dieser Periode gleich ist:
- einer ersten Messperiode, wenn der Rumpf der Person nicht vertikal ist; und/oder
- einer zweiten Messperiode, die kürzer ist als der erste Wert, wenn der Rumpf der Person vertikal ist; und
- einer dritten Messperiode, die kürzer ist als der zweite Wert, wenn die Person geht.

14. Eichverfahren einer Hilfsvorrichtung gemäß einem der voranstehenden Ansprüche, umfassend die Schritte, gemäß denen:
- der Beschleunigungsmesser auf dem Rumpf der Person angeordnet ist,
- eine Serie von Beschleunigungsmessungen erworben ist, während die Person geht,
- die Teile der Serie von Beschleunigungsmessungen, die einer beeinträchtigten Motorik der Person entsprechen, aus der Serie ausgeschlossen sind,
- die Grenzwerte einer erwarteten Beschleunigung und/oder der Warn-Grenzwert einer Beschleunigung ausgehend von Beschleunigungen der ausgeschlossenen Serie bestimmt sind.

15. Eichverfahren gemäß dem voranstehenden Anspruch, bei dem der Gang-Grenzwert der Beschleunigung und/oder der Zeit-Grenzwert ausgehend von dem Mittel der Beschleunigungen der ausgeschlossenen Serie bestimmt ist/sind.

## Claims

1. An assistance device for a person (1) suffering from motor disorders of neurological origin likely to cause freezing of gait, comprising:
• an accelerometer adapted to be worn by the person (1) to measure accelerations along at least one first direction which corresponds to the horizontal axis; and
• means (5) for emitting an auditory stimulation perceptible by the person wearing the accelerometer; and
• calculation means connected to the accelerometer, to storage means and to the emission means;
the storage means storing a plurality of anticipatory acceleration threshold values previously defined for said first direction, each anticipatory acceleration threshold value being associated with a posture, and the calculation means being configured to:
a) determine a posture of the person from the measured accelerations,
b) compare the accelerations measured by said accelerometer in the first direction relative to at least one anticipatory acceleration threshold value associated with said posture;
c) control emission of an auditory stimulation based on said anticipatory acceleration threshold value associated with said posture being crossed;
the assistance device being **characterised in that** the calculation means are configured to:
- also associate each posture with a determined period of time being a function of the crossing sense of said anticipatory acceleration threshold value by the measured accelerations, and
- control emission of the auditory stimulation based on said anticipatory acceleration threshold value associated with said posture being crossed during the determined period of time.

2. The device according to the preceding claim, wherein the posture is a seated-lying posture, and an anticipatory acceleration threshold value associated with the seated-lying posture corresponds to a backward loss of balance, the calculation means being configured to control emission of the auditory stimulation when the measured accelerations are greater than said anticipatory acceleration threshold value associated with the seated-lying posture corresponding to a backward loss of balance.

3. The device according to one of the preceding claims, wherein the posture is a standing posture, an anticipatory acceleration threshold value associated with the standing posture corresponding to a backward loss of balance and an anticipatory acceleration threshold value associated with the standing posture corresponding to a forward movement, the calculation means being configured to control emission of the auditory stimulation when the measured accelerations are lower than the anticipatory acceleration threshold value associated with the standing posture corresponding to a backward loss of balance and when the measured accelerations are greater than the anticipatory acceleration threshold value associated with the standing posture corresponding to a forward movement.

4. The device according to one of the preceding claims, comprising an inertial unit of which the accelerometer is part, said inertial unit being adapted to be worn on the person's trunk and being further configured to determine an orientation of the person's trunk, said orientation being used to determine the person's posture.

5. The assistance device according to one of the preceding claims, wherein the calculation means are configured to control emission means to emit the auditory stimulation according to a reference period corresponding to a calculated frequency of the person's steps.

6. The device according to one of the preceding claims, wherein the accelerometer is also adapted to measure accelerations along a second direction, said second direction corresponding to the vertical, and the storage means store at least one alert acceleration threshold value previously defined in a second direction, the calculation means being further configured to
a) compare the accelerations measured in the second direction relative to said at least one alert acceleration threshold value,
b) generate an alert signal when a measured acceleration has a value greater than the alert acceleration threshold value;
c) control emission means to emit an auditory stimulation based on the alert signal.

7. The device according to the preceding claim, **characterised in that** the storage means store a time threshold value and a walking acceleration threshold value, and **in that** the calculation means are configured to measure a time interval between two acceleration measurements exceeding said walking acceleration threshold value and trigger an alert when a time interval between two consecutive exceedances of said walking acceleration threshold value is lower than the time threshold value stored.

8. The assistance device according to one of claims 6 to 7, wherein the storage means store a reference period corresponding to the time threshold value, and the calculation means are configured to control emission means to emit the auditory stimulation according to the reference period.

9. The assistance device according to one of claims 6 to 7, **characterised in that** the calculation means are configured to control emission means to emit an auditory stimulation from the time when at least two alert signals are generated in a time interval lower than 0.5 second, preferably lower than 0.3 second.

10. The assistance device according to one of the preceding claims, **characterised in that** it comprises:
- means for measuring the heart rhythm adapted to be worn by the person to measure the heart rhythm thereof; and **in that**
- the storage means store a heart rhythm threshold value;
and **in that** the calculation means are configured to generate an alert signal when the measured heart rhythm has a value greater than a heart rhythm threshold value stored.

11. The assistance device according to one of the depending claims, wherein the accelerometer is configured to measure an acceleration along another direction different from that of the second direction, and the storage means store a second alert acceleration threshold value associated with said other direction, and the calculation means are configured to inhibit the generation of an alert signal during a predetermined period of time when an acceleration measured in said other direction has a value greater than the second acceleration threshold value stored.

12. The assistance device according to the preceding claims, wherein the storage means store a walking acceleration threshold value and a rest time value, and the calculation means are configured to detect a seated or lying position of the person wearing the accelerometer when all the acceleration values measured by the accelerometer are lower than the walking acceleration threshold value during a period of time greater than the rest time value.

13. The assistance device according to one of both preceding claims, **characterised in that** the calculation means are configured to compare the accelerations measured by the accelerometers periodically, the value of this period being equal to:
- a first measurement period when the person's trunk is non-vertical; and/or
- a second measurement period lower than the first value, when the person's trunk is vertical; and
- a third measurement period lower than the second value, when the person is walking.

14. A method for calibrating an assistance device according to one of the preceding claims, comprising steps in which:
- the accelerometer is disposed on the person's trunk,
- a series of acceleration measurements is acquired while the person is walking,
- the series is truncated of parts of the series of acceleration measurements corresponding to an impaired motricity of the person,
- the anticipatory acceleration threshold values and/or the alert acceleration threshold value are determined from the accelerations of the truncated series.

15. The calibration method according to the preceding claim, wherein the walking acceleration threshold value and/or the time threshold value are determined from the average of the accelerations of the truncated series.
